(19) Europäisches Patentamt
European Patent Office
Office européen des brevets

(11) **EP 4 172 618 B1**

(12) **EUROPÄISCHE PATENTSCHRIFT**

(45) Veröffentlichungstag und Bekanntmachung des Hinweises auf die Patenterteilung:
**27.08.2025 Patentblatt 2025/35**

(21) Anmeldenummer: **21729459.4**

(22) Anmeldetag: **25.05.2021**

(51) Internationale Patentklassifikation (IPC):
***G01N 33/00*** *(2006.01)*

(52) Gemeinsame Patentklassifikation (CPC):
**G01N 33/0036**

(86) Internationale Anmeldenummer:
**PCT/EP2021/063924**

(87) Internationale Veröffentlichungsnummer:
**WO 2021/259581 (30.12.2021 Gazette 2021/52)**

(54) **VERFAHREN ZUR TEMPERATURABHÄNGIGEN GASDETEKTION MIT EINER GASSELEKTIVEN MEMBRAN**

METHOD FOR THE TEMPERATURE-DEPENDENT DETECTION OF GAS USING A GAS-SELECTIVE MEMBRANE

PROCÉDÉ DE DÉTECTION DE GAZ DÉPENDANT DE LA TEMPÉRATURE À L'AIDE D'UNE MEMBRANE À SÉLECTIVITÉ GAZEUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.06.2020 DE 102020116660**

(43) Veröffentlichungstag der Anmeldung:
**03.05.2023 Patentblatt 2023/18**

(73) Patentinhaber: **Inficon GmbH**
**50968 Köln (DE)**

(72) Erfinder: **DECKER, Silvio**
**50968 Köln (DE)**

(74) Vertreter: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) Entgegenhaltungen:
EP-B1- 1 250 604          DE-A1- 10 000 496
DE-A1- 102013 227 014     DE-A1- 102016 200 270
US-A- 3 280 619           US-A1- 2011 228 276

• **AKIHIKO TANIOKA: "Temperature and Pressure Dependence of Gas Permeability through Inhomogeneous Polymer Membranes (Commemoration Issue Dedicated to Professor Hisashi Odani On the Occasion of His Retirement)", BULLETIN OF THE INSTITUTE FOR CHEMICAL RESEARCH, KYOTO UNIVERSITY, 30 September 1992 (1992-09-30), pages 178 - 187, XP055608594, Retrieved from the Internet <URL:https://core.ac.uk/download/pdf/39209998.pdf>**

**Beschreibung**

Die Erfindung betrifft ein Verfahren zur Gasdetektion mit einer gasselektiven Membran, deren Permeabilität für ein bestimmtes Gas temperaturabhängig ist.

Es ist bekannt, zur Gasdetektion mit derartigen gasselektiven Membranen die Membrantemperatur auf einen Temperaturwert einzustellen, bei dem die Gaspermeabilität der Membran für den zu detektierenden Gastypen maximal ist. Hierzu wird die Membran typischerweise beheizt, wenn die Gaspermeabilität bei einer Temperatur, die größer ist als Zimmertemperatur, maximal ist.

Die Menge des durch die Membran permeierenden Gases hängt von der Temperatur der Membran und von der Partialdruckdifferenz des Gases stromaufwärts, d.h. vor der Membran, und des Gases stromabwärts, d.h. hinter der Membran, ab. Der Nachweis des zu detektierenden Gases stromabwärts der Membran erfolgt mit Hilfe eines Detektors, bei dem es sich um eine Gasmessvorrichtung, wie z.B. ein Massenspektrometer, oder um eine Druckmessvorrichtung, die den Totaldruck des Gases misst, handeln kann. Eine Totaldruckmessung des Gases ist von Vorteil, wenn die Membran für einen bestimmten Gastypen hochselektiv ist.

Bei der temperaturabhängigen Gasdetektion mit Hilfe einer gasselektiven Membran wird das Messsignal des Gases von einem Offset-Signal überlagert, das z.B. von der Temperatur oder dem Alter des Gassensors abhängt. Um das Offset-Signal zu ermitteln, kann das Gas stromaufwärts des Sensors durch Evakuierung mit Hilfe von Pumpen entfernt werden, um das Offset-Signal im Vakuum ohne Gas zu messen. Aus DE102016200270 ist bekannt, eine offsetreduzierte Gasdetektion mit Hilfe von zwei Membranen durchzuführen, wobei die eine dem Messgas ausgesetzt ist und die andere nicht.

Aus Akihiko Tanioka: "Temperature and Pressure Dependence of Gas Permeability through Inhomogeneous Polymer Membranes",Bulletin of the Institute for Chemical Research, 1992, S.178-187, sind Membranen bekannt, die bei unterschiedlichen Temperaturen eine unterschiedliche Gasdurchlässigkeit für bestimmte Gase aufweisen.

Der Erfindung liegt die Aufgabe zugrunde, ein Verfahren zur Gasdetektion mit einer gasselektiven Membran zu schaffen, bei dem der Einfluss des Offset-Signals auf das Messsignal reduziert ist.

Das erfindungsgemäße Verfahren wird definiert durch die Merkmale von Patentanspruch 1.

Die erfindungsgemäße Gasdetektion erfolgt mit Hilfe einer gasselektiven Membran mit temperaturabhängiger Permeabilität für das zu detektierende Gas. Eine Temperaturvorrichtung ist dazu ausgebildet, die Temperatur der Membran zu verändern. Bei der Temperaturvorrichtung handelt es sich vorzugsweise um eine Heizung. Eine Kühlvorrichtung ist jedoch ebenfalls denkbar, wenn die Permeabilität der Membran bei einer Temperatur, die geringer ist als die Umgebungstemperatur der Membran, maximal ist. Ein Detektor ist dazu vorgesehen und ausgebildet, in Abhängigkeit von der Menge des durch die Membran hindurchtretenden Gases ein Messsignal zu erfassen. Bei dem Detektor kann es sich um eine Gasmessvorrichtung, wie z.B. ein Massenspektrometer, oder um eine Druckmessvorrichtung, wie z.B. um eine Totaldruckmessvorrichtung, handeln.

Nach dem erfindungsgemäßen Verfahren wird die Temperatur der Membran mit der Temperaturvorrichtung verändert, um die Membrantemperatur in zeitlicher Reihenfolge nacheinander auf mindestens zwei verschiedene Werte einzustellen, die ungleich Null sind. Aus dem Messsignal des Detektors wird mindestens ein erster Messwert zu einem ersten Zeitpunkt erfasst, an dem die Membrantemperatur einen ersten Temperaturwert einnimmt. Anschließend wird zu einem zweiten, von dem ersten verschiedenen Zeitpunkt mindestens ein zweiter Messwert des Messsignals erfasst, wobei die Membrantemperatur zu dem zweiten Zeitpunkt einen von dem ersten Temperaturwert verschiedenen zweiten Temperaturwert einnimmt. Aus den beiden erfassten Messwerten wird die Differenz gebildet. Die Beurteilung, ob ein zu detektierendes Gas vorliegt und gemessen wurde, erfolgt anhand des gebildeten Differenzsignals.

Die Permeabilität der gasselektiven Membran sollte für das zu detektierende Gas bei einer der beiden Membrantemperaturen größer sein als bei der anderen Membrantemperatur. Zum Beispiel kann die Permeabilität bei dem ersten Temperaturwert der Membrantemperatur größer sein als bei dem zweiten Temperaturwert. Vorzugsweise ist die Permeabilität bei dem ersten Temperaturwert maximal.

Wenn die Permeabilität der Membran bei der zweiten Membrantemperatur geringer ist als bei der ersten Membrantemperatur, ist der Anteil des Offsets in dem zweiten Messwert geringer als in dem ersten Messwert. In dem ersten Messwert ist hingegen derjenige Anteil des Messsignals größer, der aufgrund der erhöhten Permeabilität durch das zu detektierende Gas erzeugt wird. Aufgrund der Differenzbildung wird der Offset-Anteil reduziert.

Vorzugsweise erfolgt die Veränderung der Membrantemperatur periodisch derart, dass die Membrantemperatur die beiden Temperaturwerte abwechselnd nacheinander in periodisch wiederkehrenden Intervallen einnimmt, wobei die Messwerte zu den jeweiligen Temperaturen in mindestens zwei verschiedenen Intervallen aufgenommen werden. Der erste Messwert wird also in nachfolgenden Intervallen jeweils dann aufgenommen, wenn die Membrantemperatur den ersten Temperaturwert einnimmt. Entsprechend wird der zweite Messwert in aufeinanderfolgenden Intervallen jeweils dann aufgenommen, wenn die Membrantemperatur den zweiten Temperaturwert einnimmt.

Das Verändern der Membrantemperatur kann in Form einer Regelung zwischen zwei Temperaturwerten ungleich null erfolgen. Die Temperaturregelung kann dadurch erfolgen, dass unter Verwendung einer Temperatur-Messvorrichtung die Membrantemperatur gemessen wird und mithilfe einer Heizvorrichtung die Membran aufgeheizt wird, um die Temperatur der Membran von einem niedrigeren Temperaturwert auf einen höheren Temperaturwert zu regeln.

Bei der Differenzbildung für die beiden Messwerte nachfolgender Intervalle können jeweils Mittelwerte der Messwerte über nachfolgende Intervalle gebildet und verwendet werden. Beispielsweise kann die Differenz aus dem Mittelwert der ersten Messwerte mindestens zwei verschiedener Intervalle und dem zwischen diesen beiden ersten Messwerten liegenden zweiten Messwerten gebildet werden. Alternativ kann die Differenz auch aus dem Mittelwert der zweiten Messwerte mindestens zwei verschiedener Intervalle und dem zwischen den beiden zweiten Messwerten liegenden ersten Messwert gebildet werden.

Zum Verändern der Temperatur der Membran kann an der Temperaturvorrichtung ein die Membrantemperatur beeinflussender Parameter eingestellt und verändert werden. Das Verändern dieses Parameters zur resultierenden Veränderung der Membrantemperatur von einem Temperaturwert zum nächsten Temperaturwert sollte erst nach einer Zeitdauer von mindestens 2 Sekunden und vorzugsweise nach einer Zeitdauer im Bereich zwischen ca. 5 und 15 Sekunden erfolgen.

Die Differenz zwischen den verschiedenen Temperaturwerten der Membrantemperatur, die mit Hilfe der Temperaturvorrichtung eingestellt und verändert werden, sollte zwischen ca. 2 K und 10 K und vorzugsweise zwischen ca. 3 K und 6 K betragen. Dies gilt insbesondere für die Differenz zwischen dem ersten Temperaturwert und dem zweiten Temperaturwert. Dabei ist die Temperaturvorrichtung vorzugsweise als Heizung ausgebildet, wobei mindestens der erste Temperaturwert der Membrantemperatur größer ist als die Temperatur der Umgebung der Membran. Vor dem Einstellen eines nachfolgenden, neuen Temperaturwerts der Membrantemperatur kann die Heizung für wenige Sekunden ausgeschaltet werden, wobei der eingestellte erste Temperaturwert und der eingestellte zweite Temperaturwert der Membrantemperatur jeweils über der Umgebungstemperatur der Membran liegen. Die Messwerte des Messsignals werden vorteilhafterweise in jedem betroffenen Intervall erst nach einigen Sekunden, vorzugsweise nach etwa 2 bis 5 Sekunden erfasst, nachdem an der Temperaturvorrichtung einer neuer Temperaturwert eingestellt bzw. der die Membrantemperatur beeinflussende Parameter verändert wurde.

Bei dem erfindungsgemäßen Verfahren wird vorzugsweise keine Pumpe verwendet, um eine Druckdifferenz zwischen dem Druck des Gases stromaufwärts der Membran und dem Druck des Gases stromabwärts der Membran zu erzeugen. Das erfindungsgemäße Verfahren kann insbesondere zur Detektion eines Gases in einem Raum eines Gebäudes verwendet werden.

Im Folgenden wird anhand der Figuren ein Ausführungsbeispiel der Erfindung näher erläutert. Es zeigen:

Figur 1     eine schematische Darstellung des Gasdetektors,

Figur 2     eine schematische Darstellung des Messsignals und

Figur 3     eine schematische Darstellung der Temperatur- und Steuervorrichtungen gemäß Fig. 1.

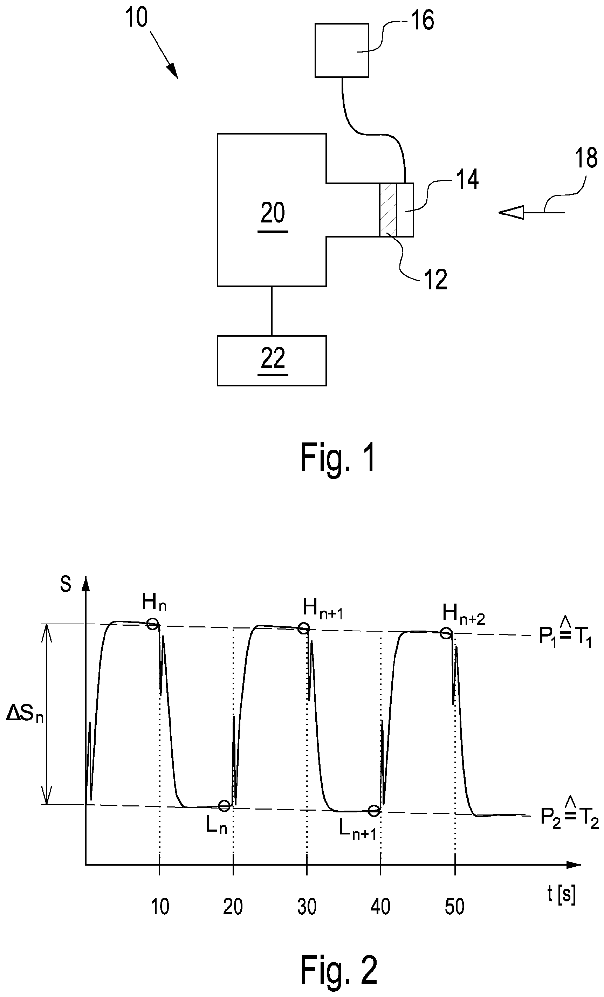

In Fig. 1 ist schematisch die Gasdetektionsvorrichtung 10 mit einer gasselektiven Membran 12 mit temperaturabhängiger Permeabilität dargestellt. Die Membran 12 wird von einer Temperaturvorrichtung 14 in Form einer Heizung mit auf der Membran 12 angeordneten Heizelementen beheizt. Über eine Steuervorrichtung 16 wird die Heizung gesteuert. Die Steuervorrichtung 16 ist Teil der Temperaturvorrichtung 14. Über die Steuervorrichtung 16 werden Parameter eingegeben, die die Leistung der Heizung und damit die Heizwirkung auf die Membran 12 beeinflussen. Dadurch kann über die Steuervorrichtung 16 ein Parameter verändert werden, der die Heizung 14 beeinflusst und die Temperatur der Membran 12 verändert. Die Heizung 14 ist derart an der Membran 12 angebracht, dass Gas aus Richtung des Pfeils 18 in Fig. 1 durch die Heizung 14 und durch die Membran 12 hindurch gelangen kann. In Bezug auf die Strömungsrichtung gemäß dem Pfeil 18 in Fig. 1 ist stromabwärts, d.h. hinter der Membran 12 ein Detektor 20 in Form einer Druckmessvorrichtung angeordnet. Durch die Membran 12 permeierendes Gas gelangt in den Detektor und erhöht dort den gemessenen Druck. Alternativ kann es sich bei dem Detektor 20 auch um einen Gasdetektor, z.B. in Form eines Massenspektrometers, handeln.

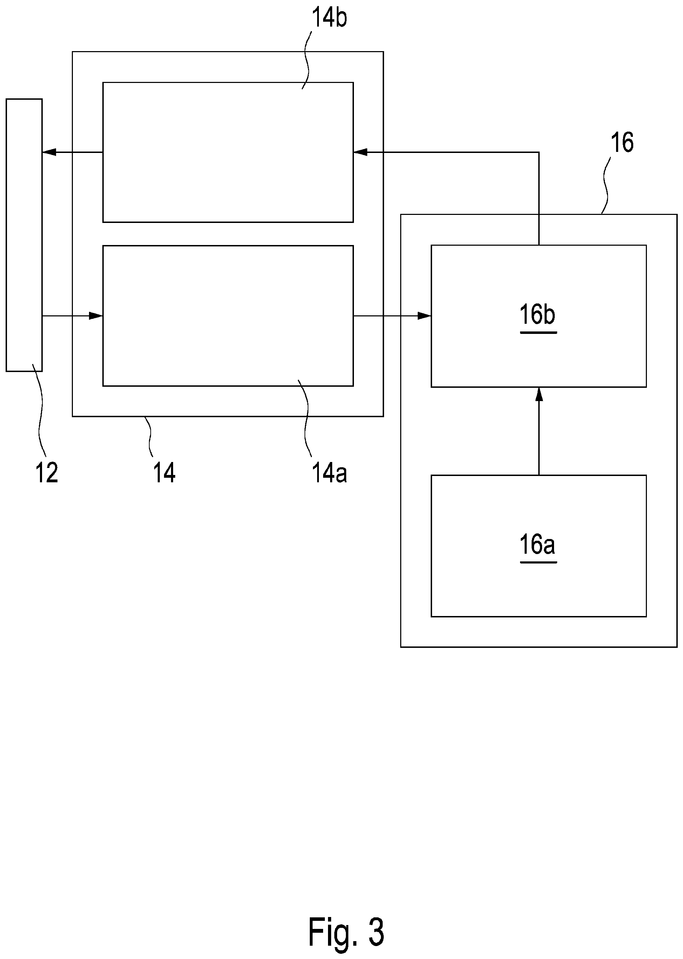

In Fig. 3 ist erkennbar, dass die Temperaturvorrichtung 14 einen Temperatursensor 14a zum Messen der Membrantemperatur und eine Membranheizung 14b zum Heizen der Membran 12 aufweist und dass die Steuervorrichtung 16 eine Temperaturvorgabe-Einrichtung 16a zum Eingeben der über die Membranheizung 14b einzustellenden Membrantemperatur und eine Regellogik 16b aufweist, die in Abhängigkeit von der mit dem Temperatursensor 14a gemessenen Membrantemperatur und der über die Temperaturvorgabe-Einrichtung 16a vorgegebenen Membrantemperatur die Heizleistung der Membranheizung 14b regelt.

Der Detektor 20 ist mit einer Auswerteeinheit 22 verbunden, die das Messsignal des Detektors 20 aufnimmt und auswertet. Mit Hilfe der Auswertevorrichtung 22 werden die Messwerte des von dem Detektor 20 generierten Messsignals erfasst und die erfindungsgemäße Differenzbildung von Messwerten ausgeführt.

In Fig. 2 ist das Messsignal S des Detektors 20 über der Zeit t in Sekunden aufgetragen. Zum Zeitpunkt t = 0 wird über die Steuervorrichtung 16 die Heizung 14 aktiviert und ein erster Parameter $P_1$ vorgegeben, der dazu führt, dass die Heizung 14 die Membran 12 auf eine erste Membrantemperatur $T_1$ aufheizt, die größer ist als die Zimmertemperatur in der Umgebung der Gasdetektionsvorrichtung 10. Beispielsweise kann die Temperatur $T_1$ 80 °C betragen. Bei der Temperatur $T_1$ handelt es sich um die Temperatur, bei der die Membran 12 eine vergleichsweise hohe Durchlässigkeit für das zu detektierende Gas, in dem vorliegenden Ausführungsbeispiel Helium, aufweist.

Infolgedessen steigt das Messsignal S, beispielsweise in Form eines elektrischen Messstromes, wie in Fig. 2 dargestellt, auf einen Maximalwert an, der zu einem Zeitpunkt t von ungefähr 10 Sekunden als erster Messwert $H_n$ gemessen wird. Anschließend wird über die Steuervorrichtung 16 der Parameter der Heizvorrichtung 14 von $P_1$ zu $P_2$ verändert, was zur Folge hat, dass die Membrantemperatur von dem Wert $T_1$ auf den Wert $T_2$ abfällt. Die zweite Membrantemperatur $T_2$ ist geringer als die erste Membrantemperatur $T_1$ und liegt dennoch oberhalb der Zimmertemperatur in der Umgebung der Gasdetektionsvorrichtung 10. Insbesondere ist die zweite Membrantemperatur $T_2$ eine Temperatur, bei der die Permeabilität der Membran 12 für das zu detektierende Gas geringer ist als bei $T_1$.

Daher fällt das Messsignal S von seinem lokalen Maximum aufgrund der Temperatur $T_1$ auf ein lokales Minimum aufgrund der Temperatur $T_2$ und wird zu einem Zeitpunkt t von ungefähr 20 Sekunden als zweiter Messwert $L_n$ gemessen. Anschließend wird über die Steuervorrichtung 16 der Parameter von $P_2$ wieder zu $P_1$ geändert und damit ein weiterer Zyklus der periodischen Wiederholung der Temperaturveränderung begonnen. Das resultierende erste Intervall wird in Fig. 2 als n bezeichnet und erstreckt sich von 0 bis zu einem Zeitpunkt t von 20 Sekunden. Zum Zeitpunkt t von 20 Sekunden beginnt dann das nachfolgende Intervall n+1 der periodischen Wiederholung der Temperaturumschaltung. Das Verändern des Parameters der Steuervorrichtung 16 von $P_2$ zu $P_1$ resultiert in einem Anstieg der Membrantemperatur hin zu der ersten Membrantemperatur $T_1$. Zu einem Zeitpunkt von ungefähr 30 Sekunden wird dann der erste Messwert des zweiten Intervalls n+1 als $H_{n+1}$ gemessen, bevor der Parameter wieder zu $P_2$ verändert und infolgedessen die Membrantemperatur auf $T_2$ abgesenkt wird. Das resultierende Messsignal wird als zweiter Messwert $L_{n+1}$ des zweiten Intervalls n+1 gemessen. Das zweite Intervall endet dann bei 40 Sekunden und das dritte Intervall n+2 beginnt.

Eine lineare Drift des Offsets des Messsignals kann wie folgt eliminiert werden:
Die ersten Messwerte $H_n$, $H_{n+1}$, $H_{n+2}$ usw. und die zweiten Messwerte $L_n$, $L_{n+1}$ usw. der jeweiligen Intervalle n, n+1, n+2 usw. werden von der Auswertevorrichtung 22 erfasst. Die Auswertevorrichtung 22 bildet eine Differenz aus dem ersten Messwert und dem zweiten Messwert, wobei bei dem vorliegenden Ausführungsbeispiel aus

den ersten Messwerten $H_n$, $H_{n+1}$ nachfolgender Intervalle n, n+1 der Mittelwert als $(H_n + H_{n+1})/2$ gebildet wird und von diesem Mittelwert der zwischen den beiden ersten Messwerten $H_n$, $H_{n+1}$ aufgenommene zweite Messwert $L_n$ subtrahiert wird. Daraus ergibt sich das Differenzsignal $\Delta S_n$ eines Intervalls n, wobei n eine natürliche Zahl größer als 0 ist, als: $\Delta S_n = (H_n + H_{n+1})/2 - L_n$.

Alternativ ist auch denkbar, dass die Mittelwertbildung aus den zweiten Messwerten $L_n$, $L_{n+1}$ zweier aufeinanderfolgender Intervalle n, n+1 als $(L_n + L_{n+1})/2$ erfolgen kann, wobei der erste Messwert für die Differenzbildung als derjenige erste Messwert $H_{n+1}$ genommen wird, der zwischen den beiden zweiten Messwerten $L_n$, $L_{n+1}$ erfasst wurde. Dabei resultiert als Signaldifferenz

$$\Delta S_n = H_{n+1} - (L_n + L_{n+1})/2.$$

Grundsätzlich ist auch denkbar, dass die Mittelwerte sowohl des ersten als auch des zweiten Messwertes gebildet werden können, wobei die Bildung der Mittelwerte auch jeweils über zwei oder mehr Intervalle erfolgen kann.

Der zweite Messwert $L_n$, $L_{n+1}$ wird in jedem Intervall zu einem Zeitpunkt aufgenommen, zu dem die Membrantemperatur auf die zweite Temperatur $T_2$ eingestellt wurde und dadurch die Permeabilität der Membran 12 minimal ist. Damit entspricht der zweite Messwert $L_n$, $L_{n+1}$ jeweils ungefähr dem Offset-Signal, welches nicht aus einem durch die Membran 12 hindurch gelangten Gas resultiert.

Die Auswerteeinheit 22 nimmt eine Beurteilung, ob das Messsignal S aus einem zu detektierenden Gas resultiert und in welcher Menge dieses Gas vorliegt, anhand des Differenzsignals $\Delta S_n$ vor, weil der Einfluss des Offset-Signals auf das Differenzsignal reduziert ist.

Die Regelung der Membrantemperatur über die Steuervorrichtung 16 und die Heizung 14 erfolgt, indem zunächst die Membrantemperatur mithilfe einer in den Figuren nicht dargestellten Messvorrichtung gemessen wird. In Abhängigkeit von dem Temperatur-Messergebnis veranlasst die Steuervorrichtung 16 ein Aufheizen der Membran 12 mithilfe der Heizung 14, bis der höhere, gewünschte Temperaturwert erreicht ist.

Die Erfindung basiert somit auf dem Prinzip eines periodischen Änderns der Membrantemperatur, um das Offset-Signal von einem aus einem detektierten Gas resultierenden Nutzsignal zu unterscheiden. Dadurch wird die Menge an Gas, die bei vorgegebener Partialdruckdifferenz der Gasdrücke vor und hinter der Membran durch die Membran hindurchgelangt, periodisch verändert. Die Signaldifferenz im Messsignal zwischen zwei Temperaturniveaus $T_1$, $T_2$ ist dabei proportional zur Partialdruckdifferenz und unabhängig von dem Sensor-Offset. Wird die Signaldifferenz gemessen und das System daraufhin kalibriert, wird das gesuchte Nutz-

EP 4 172 618 B1

signal unabhängig von dem störenden Offset-Signal des Sensors erhalten.

Die Differenz zwischen den beiden Temperaturniveaus $T_1$, $T_2$ beträgt in dem vorliegenden Ausführungsbeispiel lediglich 5 K. Der Signalhub $\Delta S$ zwischen den beiden Temperaturniveaus kann aus der Mittelung zweier benachbarter Signale hoher Temperatur, $H_n + H_{n+1}$, und dem dazwischenliegenden Signal des niedrigeren Temperaturniveaus $L_n$ gewonnen werden. Prinzipiell ist es auch möglich, das Signal aus der Mittelung zweier niedriger Niveaus und dem dazwischenliegenden mittleren hohen Niveau zu gewinnen. Auf diese Weise wird auch eine lineare Drift des Untergrundes kompensiert. Die Drift ist in Fig. 2 als negative Steigung der von links nach rechts abfallenden gestrichelten Linien der Signalamplituden erkennbar.

Das erfindungsgemäße Verfahren wird vorzugsweise zur Raumluftüberwachung eingesetzt. Dabei muss der Sensor nicht zwingend schnell reagieren. Eine aktive Förderung von Luft ist ebenfalls nicht zwingend erforderlich.

Die Membrantemperatur $T_1$, $T_2$ wird variiert, um die Permeabilität der Membran 12 und damit die Sensorempfindlichkeit der Gasdetektionsvorrichtung 10 zu verändern. Diese Änderung der Empfindlichkeit geht mit einer Änderung des Messsignals S, vorliegend eine Stromänderung, einher, die proportional zum Partialdruck des zu detektierenden Gases (Helium) ist. Die Variation der Membrantemperatur wird durch Veränderung des Parameters $P_1$, $P_2$ erreicht. Die Parameter werden dabei auf möglichst niedrige Werte eingestellt, um bei atmosphärischem Druck einen möglichst kleinen dauerhaften Sensorstrom (Messsignal S) zu erzeugen, um die Lebensdauer des Sensors nicht unnötig zu reduzieren. Die Änderung des Parameters $P_1$, $P_2$ erfolgt in dem vorliegenden Ausführungsbeispiel gemäß Fig. 2 alle 10 Sekunden, wobei zwischen den Parametern $P_1$ und $P_2$ hin und her geschaltet wird. Nach Verändern des Parameters wird ca. 8 Sekunden bis zur Messung des jeweiligen Messwertes $H_n$, $L_n$ gewartet, um die für die Heizungsregelung erforderliche Zeitdauer abzuwarten.

**Patentansprüche**

1. Verfahren zur Gasdetektion mit einer gasselektiven Membran (12) mit temperaturabhängiger Permeabilität, einer Temperaturvorrichtung (14), die dazu ausgebildet ist, die Temperatur der Membran (12) zu verändern, und einem Detektor (20), der dazu ausgebildet ist, in Abhängigkeit von der Menge des durch die Membran (12) hindurchtretenden Gases ein Messsignal zu erfassen, das zum Partialdruck eines zu detektierenden Gases proportional ist, **gekennzeichnet durch** die Schritte:

   - Verändern der Temperatur der Membran (12) mit der Temperaturvorrichtung (14),
   - Erfassen mindestens eines ersten Messwertes ($H_n$, $H_{n+1}$, $H_{n+2}$) mit dem Detektor (20) zu einem Zeitpunkt (t), an dem die Membrantemperatur einen ersten Temperaturwert ($T_1$) einnimmt,
   - Erfassen mindestens eines zweiten Messwertes ($L_n$, $L_{n+1}$) mit dem Detektor (20) zu einem Zeitpunkt (t), an dem die Membrantemperatur einen zweiten, von dem ersten verschiedenen Temperaturwert ($T_2$) einnimmt, wobei die Permeabilität bei einem der beiden Temperaturwerte ($T_1$, $T_2$) größer ist als bei dem anderen Temperaturwert,
   - Bilden der Differenz aus den beiden Messwerten und
   - Beurteilen anhand der Differenz, ob ein zu detektierendes Gas vorliegt.

2. Verfahren nach Anspruch 1, **dadurch gekennzeichnet, dass** die Veränderung der Membrantemperatur periodisch derart erfolgt, dass die Membrantemperatur die beiden Temperaturwerte ($T_1$, $T_2$) abwechselnd in periodisch wiederkehrenden Intervallen (n, n+1) einnimmt, wobei die Messwerte ($H_n$, $L_n$, $H_{n+1}$, $H_{n+1}$, $L_{n+1}$) zu den jeweiligen Temperaturen während mindestens zweier verschiedener Intervalle (n, n+1) aufgenommen werden.

3. Verfahren nach Anspruch 2, **dadurch gekennzeichnet, dass** die Differenz aus dem Mittelwert der ersten Messwerte ($H_n$, $H_{n+1}$) mindestens zweier verschiedener Intervalle (n, n+1) und dem zwischen den beiden ersten Messwerten ($H_n$, $H_{n+1}$) liegenden zweiten Messwert ($L_n$) gebildet wird.

4. Verfahren nach Anspruch 2, **dadurch gekennzeichnet, dass** die Differenz aus dem Mittelwert der zweiten Messwerte ($L_n$, $L_{n+1}$) mindestens zweier verschiedener Intervalle (n, n+1) und dem zwischen den beiden zweiten Messwerten ($L_n$, $L_{n+1}$) liegenden ersten Messwert ($H_{n+1}$) gebildet wird.

5. Verfahren nach einem der vorhergehenden Ansprüche, **dadurch gekennzeichnet, dass** das Verändern der Temperatur durch Messen und Regeln der Temperatur erfolgt, wobei die Regelung der Temperatur zwischen zwei Temperaturwerten ($T_1$, $T_2$) erfolgt, die größer als Null sind.

6. Verfahren nach einem der vorhergehenden Ansprüche, **dadurch gekennzeichnet, dass** das Verändern der Temperatur von einem Temperaturwert ($T_1$) zum nächsten Temperaturwert ($T_2$) nach mindestens 2 Sekunden und vorzugsweise nach ca. mindestens 5-15 Sekunden erfolgt.

7. Verfahren nach einem der vorhergehenden Ansprüche, **dadurch gekennzeichnet, dass** die Differenz zwischen den eingestellten Temperaturwerten ($T_1$, $T_2$) der Membrantemperatur zwischen ca. 2-10 K und vorzugsweise etwa 3-6 K beträgt.

8. Verfahren nach einem der vorhergehenden Ansprüche, **dadurch gekennzeichnet, dass** die Messwerte des Messsignals (S) in jedem Intervall erst nach mindestens ca. 2-5 Sekunden erfasst werden, nachdem ein neuer Temperaturwert für die Temperaturvorrichtung (14) eingestellt wurde.

9. Verfahren nach Anspruch 8, **dadurch gekennzeichnet, dass** die Temperaturvorrichtung (14) eine Heizung ist, wobei mindestens der erste Temperaturwert ($T_1$) der Membrantemperatur größer ist als die Temperatur der Umgebung der Membran (12).

10. Verfahren nach Anspruch 9, **dadurch gekennzeichnet, dass** vor dem Einstellen eines neuen Temperaturwertes der Membrantemperatur die Heizung (14) ausgeschaltet wird, wobei der erste Temperaturwert ($T_1$) und der zweite Temperaturwert ($T_2$) jeweils über der Umgebungstemperatur liegen.

11. Verfahren nach einem der vorhergehenden Ansprüche, **dadurch gekennzeichnet, dass** keine Pumpe zum Erzeugen einer Druckdifferenz zwischen den Drücken des Gases stromaufwärts der Membran (12) und des Gases stromabwärts der Membran (12) verwendet wird.

12. Verfahren nach einem der vorhergehenden Ansprüche, **dadurch gekennzeichnet, dass** das Verfahren zur Detektion eines Gases in einem Raum eines Gebäudes verwendet wird.

13. Verfahren nach einem der vorhergehenden Ansprüche, **dadurch gekennzeichnet, dass** der Detektor (20) eine Gasmessvorrichtung oder eine Druckmessvorrichtung ist.

**Claims**

1. A method for the detection of gas using a gas-selective membrane (12) having a temperature-dependent permeability, a temperature device (14) that is designed to change the temperature of the membrane (12), and a detector (20) that is designed to detect a measurement signal on the basis of the amount of gas passing through the membrane (12), the measurement signal being proportional to the partial pressure of a gas to be detected **characterized by** the following steps:

    - changing the temperature of the membrane (12) using the temperature device (14),

    - acquiring at least one first measuring value ($H_n$, $H_{n+1}$, $H_{n+2}$) using the detector (20) at a time (t) at which the membrane temperature adopts a first temperature value ($T_1$),
    - acquiring at least one second measuring value ($L_n$, $L_{n+1}$) using the detector (20) at a time (t) at which the membrane temperature adopts a second temperature value ($T_2$) different from the first temperature value, wherein the permeability of one of the two temperature values ($T_1$, $T_2$) is greater than of the other temperature value,
    - calculating the difference between the two measuring values, and
    - using the difference to assess whether a gas to be detected is present.

2. The method according to claim 1, **characterized in that** the change of the membrane temperature is performed periodically such that the membrane temperature alternately adopts the two temperature values ($T_1$, $T_2$) at periodically recurring intervals (n, n+1), the measuring values ($H_n$, $L_n$, $H_{n+1}$, $H_{n+1}$, $L_{n+1}$) for the respective temperatures being acquired during at least two different intervals (n, n+1).

3. The method according to claim 2, **characterized in that** the difference is calculated between the means value of the first measuring values ($H_n$, $H_{n+1}$) of at least two different intervals (n, n+1) and the second measuring value ($L_n$) between the two first measuring values ($H_n$, $H_{n+1}$).

4. The method according to claim 2, **characterized in that** the difference is calculated between the means value of the second measuring values ($L_n$, $L_{n+1}$) of at least two different intervals (n, n+1), and the first measuring value ($H_{n+1}$) between the two second measuring values ($L_n$, $L_{n+1}$).

5. The method according to any one of the preceding claims, **characterized in that** changing the temperature is affected by measuring and controlling the temperature, the temperature control being performed between two temperature values ($T_1$, $T_2$) greater than zero.

6. The method according to any one of the preceding claims, **characterized in that** changing the temperature from one temperature value ($T_1$) to the next temperature value ($T_2$) is performed after at least 2 seconds and preferably after approx. at least 5-15 seconds.

7. The method according to any one of the preceding claims, **characterized in that** the difference between the set temperature values ($T_1$, $T_2$) of the membrane temperature is between approx. 2-10 K and preferably between about 3-6 K.

**8.** The method according to any one of the preceding claims, **characterized in that** the measuring values of the measuring signal (S) are acquired in each interval only after at least approx. 2-5 seconds, after a new temperature value has been set for the temperature device (14).

**9.** The method according to claim 8, **characterized in that** the temperature device (14) is a heating, wherein at least the first temperature value ($T_1$) of the membrane temperature is higher than the temperature of the environment of the membrane (12).

**10.** The method according to claim 9, **characterized in that** prior to setting a new temperature value of the membrane temperature, the heating (14) is deactivated, wherein the first temperature ($T_1$) value and the second temperature value ($T_2$) are each higher than the ambient temperature.

**11.** The method according to any one of the preceding claims, **characterized in that** no pump is used to create a differential pressure between the pressures of the gas upstream of the membrane (12) and the gas downstream of the membrane (12).

**12.** The method according to any one of the preceding claims, **characterized in that** the method is used to detect a gas in a room of a building.

**13.** The method according to any one of the preceding claims, **characterized in that** the detector (20) is a gas measuring device or a pressure measuring device.

**Revendications**

**1.** Procédé de détection de gaz à l'aide d'une membrane à sélectivité gazeuse (12) ayant une perméabilité dépendante de la température, d'un dispositif de température (14) qui est configuré pour changer la température de la membrane (12), et d'un détecteur (20) qui est configuré pour capter un signal de mesure proportionnel à la pression partielle d'un gaz à détecter, en fonction de la quantité de gaz traversant la membrane (12),
**caractérisé par** les étapes :

- changer la température de la membrane (12) à l'aide du dispositif de température (14),
- capter au moins une première valeur de mesure ($H_n$, $H_{n+1}$, $H_{n+2}$) à l'aide du détecteur (20) à un moment (t) auquel la température de la membrane atteint une première valeur de température ($T_1$),
- capter au moins une deuxième valeur de mesure ($L_n$, $L_{n+1}$) à l'aide du détecteur (20) à un moment (t) auquel la température de la membrane atteint une deuxième valeur de température ($T_2$) différente de la première, la perméabilité à une des deux valeurs de température ($T_1$, $T_2$) étant plus grande qu'à l'autre valeur de température,
- former la différence entre les deux valeurs de mesure et
- décider selon la différence si un gaz à détecter est présent.

**2.** Procédé selon la revendication 1, **caractérisé en ce que** le changement de température de la membrane est effectué périodiquement de façon telle que la température de la membrane prend les deux valeurs de température ($T_1$, $T_2$) en alternance dans des intervalles (n, n+1) périodiquement répétitifs, les valeurs de mesure ($H_n$, $L_n$, $H_{n+1}$, $H_{n+2}$, $L_{n+1}$) aux températures respectives étant captées pendant au moins deux intervalles différents (n, n+1).

**3.** Procédé selon la revendication 2, **caractérisé en ce que** la différence est formée à partir de la valeur moyenne des premières valeurs de mesure ($H_n$, $H_{n+1}$) d'au moins deux intervalles différents (n, n+1) et de la deuxième valeur de mesure ($L_n$) située entre les deux premières valeurs de mesure ($H_n$, $H_{n+1}$).

**4.** Procédé selon la revendication 2, **caractérisé en ce que** la différence est formée à partir de la valeur moyenne des deuxièmes valeurs de mesure ($L_n$, $L_{n+1}$) d'au moins deux intervalles différents (n, n+1) et de la première valeur de mesure ($H_{n+1}$) située entre les deux deuxièmes valeurs de mesure ($L_n$, $L_{n+1}$).

**5.** Procédé selon l'une des revendications précédentes, **caractérisé en ce que** le changement de température est effectué en mesurant et réglant la température, le réglage de la température étant effectué entre deux valeurs de température ($T_1$, $T_2$) qui sont supérieures à zéro.

**6.** Procédé selon l'une des revendications précédentes, **caractérisé en ce que** le changement de température d'une valeur de température ($T_1$) à la valeur de température suivante ($T_2$) est effectué après au moins 2 secondes et de préférence après environ au moins 5 à 15 secondes.

**7.** Procédé selon l'une des revendications précédentes, **caractérisé en ce que** la différence entre les valeurs de température ($T_1$, $T_2$) de la température de la membrane est entre environ 2 à 10 K et de préférence environ 3 à 6 K.

**8.** Procédé selon l'une des revendications précédentes, **caractérisé en ce que** les valeurs de mesure du signal de mesure (S) sont captées à chaque intervalle après seulement au moins environ 2 à 5 secondes après le réglage d'une nouvelle valeur de mesure pour le dispositif de température (14).

9.  Procédé selon la revendication 8, **caractérisé en ce que** le dispositif de température (14) est un chauffage, au moins la première valeur de température ($T_1$) de la température de la membrane étant supérieure à la température de l'environnement de la membrane (12).

10. Procédé selon la revendication 9, **caractérisé en ce que**, avant le réglage d'une nouvelle valeur de température de la température de la membrane, le chauffage est éteint, la première valeur de température ($T_1$) et a deuxième valeur de température ($T_2$) étant chacune supérieur à la température de l'environnement.

11. Procédé selon l'une des revendications précédentes, **caractérisé en ce qu'**aucune pompe n'est utilisée pour générer une différence de pression entre les pressions du gaz en amont de la membrane (12) et du gaz en aval de la membrane (12).

12. Procédé selon l'une des revendications précédentes, **caractérisé en ce que** le procédé pour la détection d'un gaz est utilisé dans une pièce d'un bâtiment.

13. Procédé selon l'une des revendications précédentes, **caractérisé en ce que** le détecteur (20) est un dispositif de mesure de gaz ou un dispositif de mesure de pression.

10

16

20

14

18

12

22

## Fig. 1

$S$

$H_n$

$H_{n+1}$

$H_{n+2}$

$P_1 \hat{=} T_1$

$\Delta S_n$

$L_n$

$L_{n+1}$

$P_2 \hat{=} T_2$

10   20   30   40   50   $t\,[s]$

## Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016200270

**In der Beschreibung aufgeführte Nicht-Patentliteratur**

- **AKIHIKO TANIOKA**. Temperature and Pressure Dependence of Gas Permeability through Inhomogeneous Polymer Membranes. *Bulletin of the Institute for Chemical Research*, 1992, 178-187